# EUROPEAN PATENT SPECIFICATION

(11) **EP 3 494 955 B1**
(45) Date of publication and mention of the grant of the patent: **27.01.2021**
(21) Application number: 17460074.2
(22) Date of filing: 08.12.2017
(51) Int. Cl.: A61K 9/00, A61K 31/5575, A61K 45/06, A61Q 7/00, A61P 17/00

(54) **COSMETIC COMPOSITION FOR PROMOTING HAIR GROWTH**
KOSMETISCHE ZUSAMMENSETZUNG ZUR FÖRDERUNG DES HAARWACHSTUMS
COMPOSITION COSMÉTIQUE POUR FAVORISER LA CROISSANCE CAPILLAIRE

(43) Date of publication of application: 12.06.2019
(73) Proprietor: Warszawski Uniwersytet Medyczny, 02-091 Warszawa (PL)
(72) Inventor: Koziak, Katarzyna, 02-708 Warszawa (PL); Rakowska, Adriana, 02-104 Warszawa (PL); Rudnicka, Lidia, 00-768 Warszawa (PL)
(74) Representative: LDS Lazewski Depo & Partners

(56) References cited:
- WO-A1-2009/151828
- WO-A1-2015/176161
- US-A1- 2010 105 771

## Description

### Field of the invention

The present invention relates to a composition comprising latanoprost acid, wherein the concentration of latanoprost acid is from 0.001 to 1%, such as 0.01%, 0.05%, 0.1%. The invention further relates, inter alia, to the use of the composition for preventing or reducing hair loss, and for fortifying hair or promoting hair growth.

### Background of the invention

Hair loss, also known as alopecia or baldness, is an extremely common problem that can affect both men and woman. Hair loss occurs naturally, however, it can also be promoted by numerous factors, such as hair damages physically or chemically caused by the hair wash, hair coloring, blow drying, physical or emotional stress, nutritional deficiencies, pharmaceuticals, in particular pharmaceuticals used in chemotherapy, or by numerous disorders. The known disorders that are associated with hair loss are, inter alia, androgenic alopecia, alopecia areata, chemotherapy-induced alopecia, cicatricial alopecia and telogen effluvium. When hair loss is not accompanied by re-growth, it causes baldness.

One of the most common forms of hair loss, which occurs in both men and woman, is androgenic alopecia. Over 50% of women and over 70% of man developing androgenetic alopecia after the age of 40, many of the very mild cases of androgenetic alopecia, remain free of typical clinical appearance of the disease and are considered "insufficient hair quality". These cases are not considered "pathology" and patients do not require typical pharmacological therapy.

Hairs are arranged in follicular units consisting of two, three, four and sometimes even five hairs per grouping, which can be easily assessed with trichoscopy (hair and scalp dermoscopy). Hair loss is typically demonstrated in the decrease of number of hairs in a follicular unit, which may eventually lead to loss of all hairs in the unit or particular hairs in follicular unit (in trichoscopy decrease in percentage of follicular units with three or more hairs and increase in percentage of follicular units with one hair will be observed). When all hairs in a follicular unit are loss, an empty follicular openings can be observed by trichoscopy as yellow dot. Decrease in mean hair shaft thickness can also lead to hair loss observed in clinical examination.

Various attempts have been made to prevent hair loss and to invoke re-growth of the hair, including cosmetics, pharmaceutics, vitamin supplementation, invasive surgical procedures and injections.

A wide variety of compounds have been proposed for fortifying and promoting hair growth. One of the compounds is minoxidil, which is a component of the approved drug for topical administration. Another approved drug, which is for oral administration, is finasteride. However, the search for further hair growth inducers is ongoing due to factors including safety concerns and limited efficacy.

### Subject of the invention

It has been found that latanoprost acid can effectively be used for preventing or reducing hair loss, and for fortifying hair or promoting hair growth in cosmetic and therapeutic applications.

In one embodiment of this invention, latanoprost acid is contained in a cosmetic, in another embodiment in a pharmaceutical composition. Such compositions are intended for topical administration.

The composition, used according to the present invention, may be administered at least once per day on the scalp.

In another embodiment, latanoprost acid may be co-administered with a further active agent. Coadministration means that the active ingredients are administered simultaneously or sequentially and in any order. Latanoprost acid and the further active ingredient may be in one composition or in two separate compositions.

The invention also relates to a cosmetic process comprising administration of latanoprost acid to the scalp.

### Detailed description of the invention

The present invention provides a composition comprising latanoprost acid. The composition of the present invention is a cosmetic or a pharmaceutical composition.

Latanoprost acid, (5Z)-7-{(1R,2R,3R,5S)-3,5-dihydroxy-2-[(3R)-3-hydroxy-5-phenylpentyl]cyclopentyl}hept-5-enoic acid, is a potent and highly selective prostaglandin (PG)F2a receptor antagonist of the following formula:

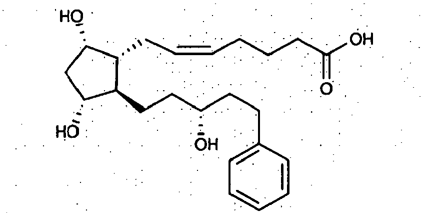

Latanoprost acid may be obtained by known methods, such as methods for obtaining presented in the following publications: *"*A new synthetic approach to high-purity (15R)-latanoprost", Martynow, J.G.; Jóźwik, J.; Szelejewski, W.; Achmatowicz, O.; Kutner, A.; Wiśniewski, K.; Winiarsk,J.; Zegrocka-Stendel, O.; Golebiewski, P., Eur. J. Org. Chem. 2007, 689-703, or in polish patent application P. 374461, *"Method for obtaining the derivatives of 13,14-dihydro-PGF₂d',* Martynow J, Jóźwik J, Szelejewski W, Achmatowicz O, Kutner A, Wiśniewski K, Winiarski J, Zegrocka-Stendel O, Go 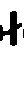 biewski P.

Latanoprost acid concentration in the composition of the invention is from 0.001 - 1%. Preferably, latanoprost acid concentration in the composition of the invention is e_{.}g. 0.01%, 0.05%, or 0.1%.

The composition of the present invention is for topical use. It may have any conventional cosmetic form, such as a cream, lotion, emulsion, solid, paste, gel, spray, stick, mouser, gel, liquid, preferably an emulsion. The composition preferably has a form of a hair care cosmetic product, such as a hair shampoo, hair mask, hair conditioner or a hair styling product. The composition may contain various components contained in conventional cosmetics, such as distilled water, thickeners, preservatives, fragrances, pigments, coloring agents, natural animal or plant oils, semi-synthetic oils, hydrocarbon oils, higher alcohol oils, ester oils, conventional silicone oils etc.

The composition of the present invention may also have any conventional pharmaceutical form and contain any conventional pharmaceutical components.

The present invention also concerns use of latanoprost acid for preventing or reducing hair loss, and for fortifying hair or for promoting hair growth. Latanoprost acid is suitable for preventing, reducing and treating hair loss in a mammal, in particular in a human, in both men and women.

No specific preparation of the skin, before application of latanoprost acid, preferably as a composition containing latanoprost acid, is required, but any specific preparation is not excluded and may yield an improvement in the positive effects of the invention. In particular, the preparation of the skin may include cleaning of the scalp. Latanoprost acid, preferably in the composition of the invention, is preferably administered to the dry skin on the scalp.

No specific procedure following the application of latanoprost acid, in particular in the composition of the invention, is required, but latanoprost acid should not be removed immediately after administration of latanoprost acid, in particular it should not be washed away or wiped. Preferably, the skin area to which latanoprost acid is applied and hair should not be contacted with water immediately after application of latanoprost acid, more preferably within 4 hours following the application, even more preferably within at least 5, 6 or 7 hours, most preferably within 8 hours following the application.

It is sufficient to apply one thin layer of the composition containing latanoprost acid on the skin, but more than one layer may also be applied, e.g. two, three or more layers.

Latanoprost acid, preferably in the composition according to the present invention should be applied at least once daily, preferably once or twice daily, for at least 1 or 2 months, preferably for at least 3 moths, more preferably for a period longer than 3 months, even more preferably for a period longer than 4 months or 5 months, and most preferably for a period of at least 6 months.

In one embodiment the invention provides therapeutic application of latanoprost acid. Preferably, latanoprost acid is administered to a mammal, in particular human, preferably to a human female. More preferably, latanoprost acid is used for treating hair loss due to female androgenic alopecia (FAGA) and/or for fortifying hair or promoting hair growth.

In accordance with the present invention there is also provided a cosmetic process of reducing or preventing or treating hair loss, which comprises the step of applying to the skin a composition containing latanoprost acid at a concentration effective to reduce or prevent or treat the said hair loss.

### Brief description of Figures

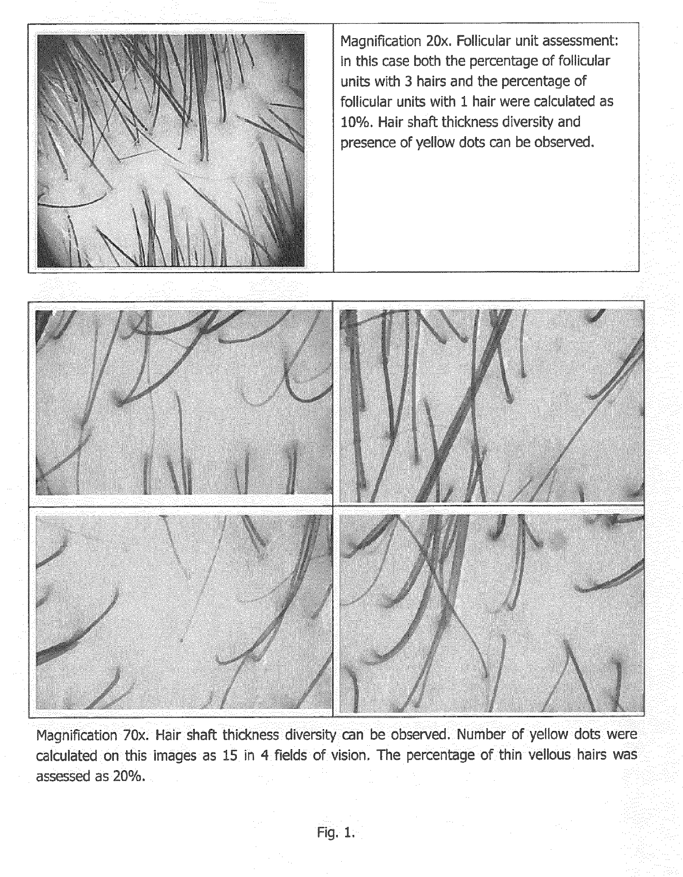

Fig. 1 shows 20-fold and 70-fold magnification of trichoscopy images used for the evaluation of trichoscopy parameters.
Fig. 2 shows clinical assessment of Ludwig scale (Ludwig's parameter: 1, 2 and 3, respectively).

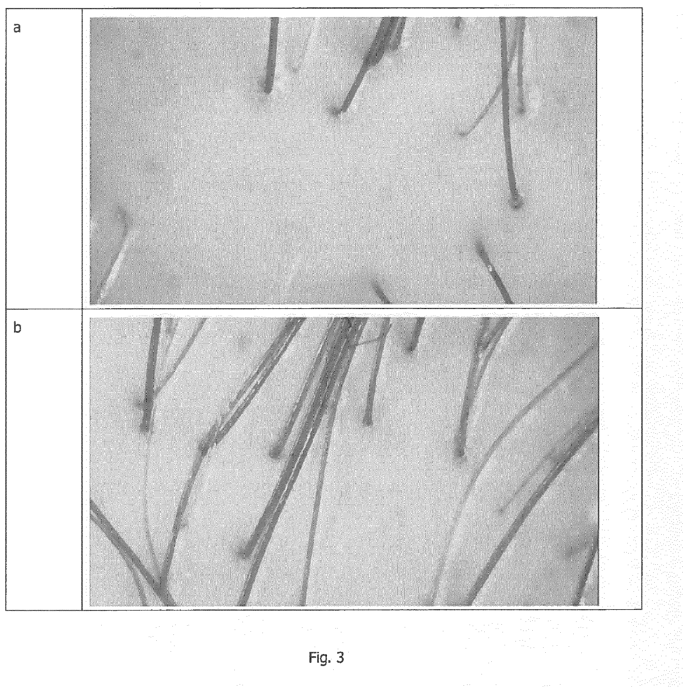

Fig. 3 shows an improvement of hair shaft thickness in a patient - Fig. 3(a) was taken before the treatment, while Fig. 3(b) was taken after 6 months of treatment with 0.1% latanoprost acid.

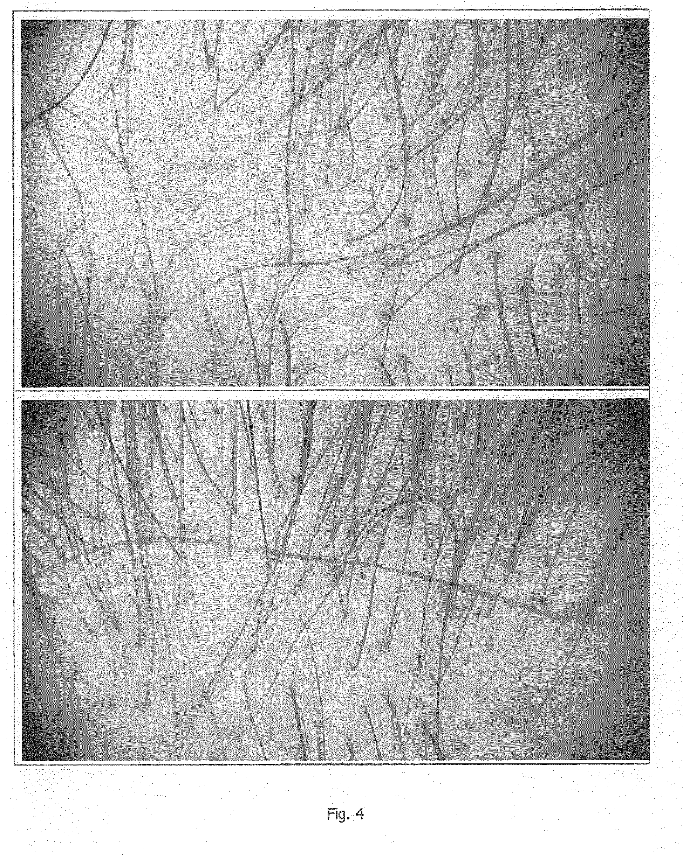

Fig. 4 shows an improvement in hair shaft thickness, in particular as an increase in percentage of follicular units with 3 hairs (5% vs 30%) and decrease in percentage of follicular units with one hair (30% vs 15%); photograph (a) was taken before and (b) after 6 months of treatment with 0.01% of latanoprost acid; magnification 20x.

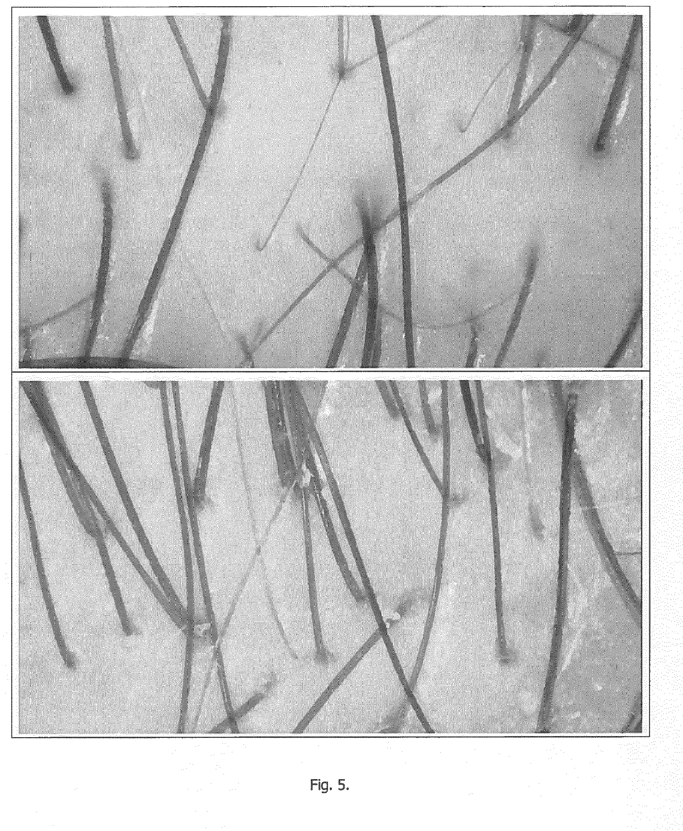

Fig. 5 shows an improvement in hair shafts thickness after 6 months of treatment with 0.05% latanoprost acid (b), as compared to baseline (a); magnification 70x.

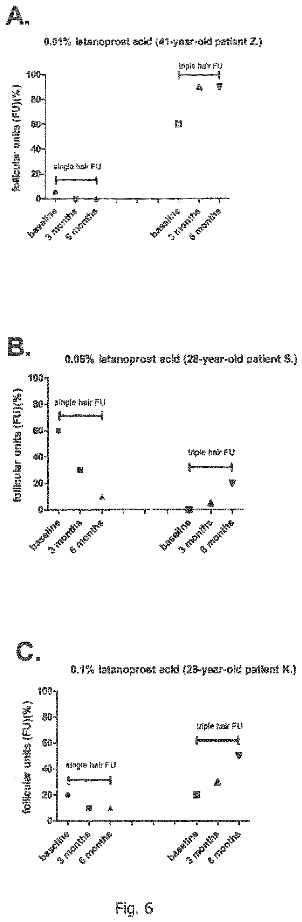

Fig. 6 shows examples of patients' response to the treatment. Depicted changes in percentage of single and triple hair follicular unit after (A) 0.01% latanoprost acid, (B) 0.05% latanoprost acid and (C) 0.1% latanoprost acid was paralleled with a decrease in a number of yellow dots which,
for the same patients, was as follow: (A) 5, 0, 0 for the baseline, 3 months and 6 months, respectively, (B) 4, 0, 3 for the baseline, 3 months and 6 months, respectively and (C) 6, 3, 3 for the baseline, 3 months and 6 months, respectively.

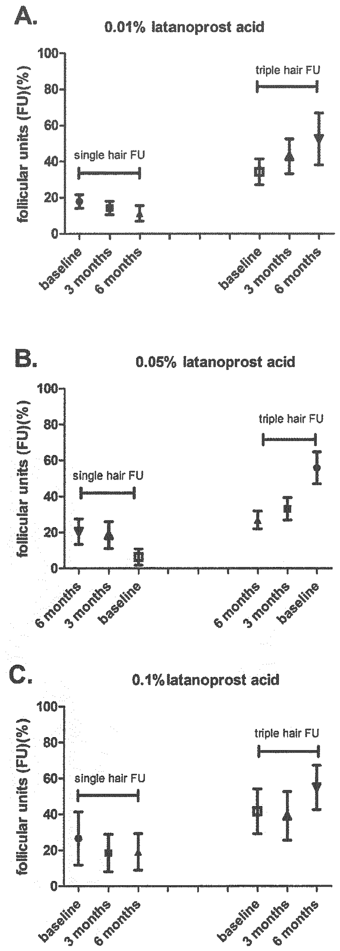

Fig. 7 shows changes in a number of follicular units with single and triple hair after 3 and 6 months of treatment with (A) 0.01% latanoprost acid, (B) 0.05% latanoprost acid and (C) 0.1% latanoprost acid.

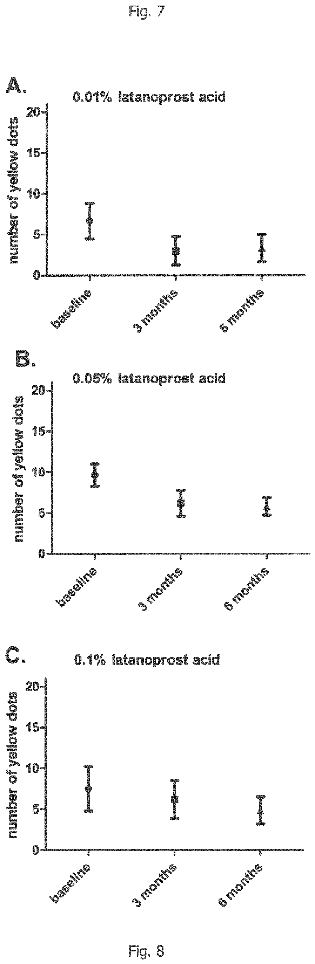

Fig. 8 shows changes in a number of yellow dots after 3 and 6 months of treatment with (A) 0.01% latanoprost acid, (B) 0.05% latanoprost acid and (C) 0.1% latanoprost acid.

### Examples

The below examples are intended to illustrate the invention and should not be interpreted as limiting.

### Example 1

A topical composition was prepared using a conventional method. The obtained composition constituted a water and paraffin emulsion. The following concentrations of latanoprost acid were obtained:

| No. | Concentration of latanoprost acid |
|---|---|
| 1 | 0 |
| 2 | 0.1 mg/g (0.01%) |
| 3 | 0.5 mg/g (0.05%) |
| 4 | 1.0 mg/g (0.1%) |

### Example 2

The study was a double blind placebo control study for which the baseline was established with trichoscopy (Videodermoscop Fotofinder Medicam 800HD, Consultronix) (random assignment of preparation). Results were gathered during clinical examination with trichoscopy after 3 and 6 months.

### PATIENTS

40 female patients (age 18-60 years) with hair loss applied to participate in the study. The exclusion criteria for participation in the study were the following conditions: alopecia areata, cicatricial alopecia and acute telogen effluvium with known cause.

29 patients were included into the study, among which 26 were diagnosed with female androgenic alopecia (FAGA) and 3 with chronic telogenum effluvium. One of the patients diagnosed with chronic telogenum effluvium had her first control visit after 3 months and the second control visit after 6 months (with significant improvement).

From the above-mentioned 26 FAGA diagnosed patients, 2 patients did not report to evaluation visit, 1 patient decided not to continue the study, 23 patients had their first control visit after 3 months, and 21 out of these 23 patients had the second evaluation visit after 6 months.

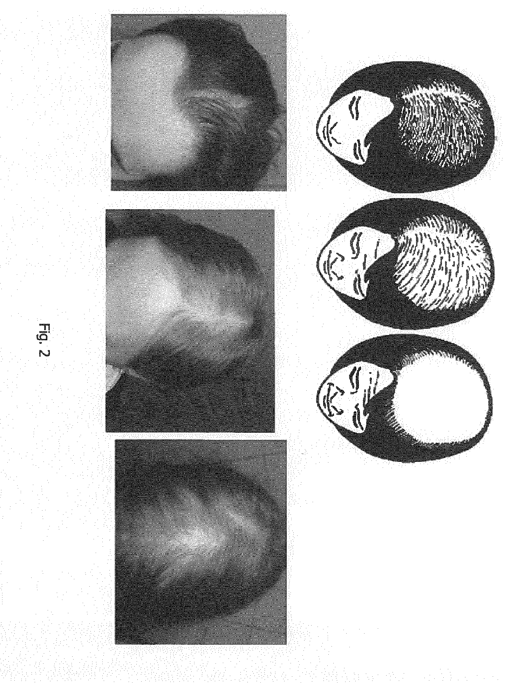

The present example refers to the analysis of the treatment of these 23 patients who had their first evaluation visit after 3 months. These patients were female patients with mean age 38 years (range 27 - 60 years). Clinical assessment of the patients at the beginning of the study included severity of the disease, classified as follows: 4 patients - Ludwig 1, 7 patients - Ludwig 2, and 5 patients - Ludwig 3 (see Fig. 2).

### STUDY DETAILS

Patients were randomly divided into 4 groups. The control group was treated with placebo (0%). Groups 2, 3 and 4 were treated with latanoprost acid composition of 0.01%, 0.05% 0.1% and latanoprost acid, respectively.

### DOSAGE REGIMEN

The composition specified in example 1 was administered topically on the scalp once daily for 3 to 6 months. The patients were instructed to apply about 2-3 ml of the composition (i.e. one dose) to the dry scalp and hair and not to wash the scalp and hair within at least 8 hours following the administration of the composition.

### DERMATOLOGICAL EXAMINATION

Patients underwent dermatological examination with trichoscopy.

### TRICHOSCOPY- EVALUATED PARAMETERS

The severity parameters were as follows:
1. Percentage of thin hairs and hair shaft heterogeneity,
2. Percentage of follicular units (FU) with single, double and triple hairs,
3. Number of yellow dots calculated in 4 images at 70-fold magnifications,
4. Peripilar sign (percentage of follicular units with this feature).

Reversing the process of androgenic alopecia may be seen in trichoscopy as:
1. Decrease in a number of yellow dots,
2. Decrease of percentage of follicular units with single hairs,
3. Increase of percentage of follicular units with three hairs

It should be noted that percentage of thin hairs (<0.03 mm) may increase at the beginning of successful treatment, because new regrowing hair are usually thin.

### RESULTS

### a) Three months of treatment

Frontal area was evaluated by trichoscopy at the beginning of the study (baseline) and 3 months later. For the evaluation purposes 1 image in 20-fold magnification and 4 images in 70-fold magnifications were made.

The percentage of follicular units with 1, 2 and 3 hairs were calculated.

### After 3 months:

- percentage of follicular units with single hair decreased by: 4% for 0.01%, 23% for 0.05% and 9% for 0.1%
- percentage of follicular units with 3 hairs increased by 9% for 0.01% and 13% for 0.05%; for 0.1% there was not a significant change.

Mean number of yellow dots in frontal area of head (calculated in 4 trichoscopic images at 70-fold magnifications) decreased significantly after 3 months by 64%, 35% and 17% for 0.01%, 0.05% and 0.1%, respectively.

Hair shafts thickness (mean diameter and percentage of thin hairs) improved in all the patients after 3 months of treatment. Using the scale from 0 to 3, where 0 means no improvement and 3 very significant improvement, in the 0.01% group all the patients were rated 1, in the 0.05% group 20% of patients were rated 1, 60% - 2 and 20% - 3, whereas in the 0. 1% group 33% of the patients received score 1 and 67% score 2.

Majority of the patients did not report any undesired effects. Only one patient receiving 0.01% treatment complained about headache and despite the observed improvement the treatment was not continued. Dermatological examination also did not reveal any side effects.

None of the evaluated parameters changed in the group receiving the placebo.

### b) Six months of treatment

### After 6 months:

- percentage of follicular units with single hair decreased by 7% for 0.01%, 29% for 0.05% and 8% for 0.1%, as compared to baseline,
- percentage of follicular units with 3 hairs increased by 18% for 0.01%, 14% for 0.05% and 13% for 0.1%, as compared to baseline.

Mean number of yellow dots in frontal area of head (calculated in 4 trichoscopic images at 70-fold magnifications) decreased significantly after 6 months by 36%, 40% and 30% for 0.01%, 0.05% and 0.1%, respectively, as compared to baseline.

No side effects were reported during interview and dermatological examination. Particular attention was given to signs and symptoms of skin irritation.

None of the evaluated parameters changed in the group receiving the placebo treatment.

### SUBJECTIVE EVALUATION

The patients were asked to provide their personal subjective evaluation regarding the results of the study:
- Group 0.01%:
   - 67% of the patients observed improvement after 3 months
   - 50% of the patients observed improvement after 6 months
- Group 0.05%:
   - 82% of the patients observed improvement after 3 months
   - 80% of the patients observed improvement after 6 months
- Group 0.1%:
   - 83% of the patients observed improvement after 3 months
   - 67% of the patients observed improvement after 6 months

### Ludwig parameter was assessed at the end of the treatment.

The following improvement in Ludwig parameters was observed after 6 months:
- Group 0.01% - improvement was observed in 50% of the patients (2 out of 4 patients)
- Group 0.05% - improvement was observed in 50% of the patients (5 out of 10 patients)
- Group 0.1% - improvement was observed in 67% of the patients (4 out of 6 patients)

### Example 3

### Skin penetration study

Domestic pig (Sus scrofa f. domestica) skin has been shown to have similar histological and physiological properties to human skin and has become an acknowledged model for studies of percutaneous penetration as a substitute for human skin. In the experiment, a suitable portion of washed and drained pig's full thickness ear skin was separated from the cartilage by a scalpel. Particular attention was given not to damage the stratum corneum. To ensure proper selection of the undamaged skin fragments, the impedance of each piece was measured. Only fragments with an impedance value of not less than 30 kΩ / cm2 (based on Ag / AgCl electrodes installed in a 12 mm diameter instrument, impedance value ≥ 27 kΩ) were used for the experiment. The selected skin discs were placed on a Franz-type diffusion cells and 1 ml of the emulsion samples containing 0,1; 0,5 or 1,0 mg/g (i.e. 0.01; 0.05 or 0.1 %) of latanoprost acid was applied. After 22 hr perception phase (pH 7.1 isotonic phosphate buffer solution), 5 ml samples were collected. The test method remains in accordance with OECD TG 428 (Skin Absorption; in vitro method). Collected samples were then subjected to chromatographic analysis.

### Chromatographic analysis

A standard of latanoprost acid bn. 0102215WS was manufactured in Pharmaceutical Research Institute, Warsaw, Poland. The HPLC grade acetonitrile, trifluoroacetic acid (HPLC grade) were purchased from Avantor Performance Materials Poland S.A. (Gliwice, Poland). Ultrapure water was obtained in this laboratory using the PureLab System Filtration (TEMPLAB, Warsaw, Poland). The Shimadzu Prominence (Shimadzu Corporation, Japan) HPLC system equipped with two LC-20AD pumps, an SIL 20-AC autosampler, a CTO-20AC column oven, an PDA-M20A photodiode array detector and a CMB-20A communication module were used. The "Lab-Solution" software (Shimadzu Corporation, Japan) was used for instrument control and data acquisition. The separation of latanoprost acid was performed on a Symmetry C18 100A (250 x 4.6 mm, 5 µm). The mobile phase consisted of a mixture of acetonitrile : water : TFA (40:60:0.017 v/v/v). The other chromatographic parameters were as follows: flow rate: 1ml min-1; column temperature: 25°C; autosampler temperature: 25°C, injection volume:20µl; UV detection wavelength: 210 nm; diluent: methanol; concentration of latanoprost acid in the investigated sample was 0.02 mg ml-1. Working standard solution of latanoprost acid was prepared by dissolving of 2 mg reference standard in a 10 ml of methyl acetate and diluting with methanol to volume 10 ml. The concentration was 0.02 mg/ml. 0.2 g of the sample emulsion was accurately weighed into a 10 ml volumetric flask and dissolved with methanol. The resultant solution was filtered through a paper filter 390 grade.

The results of the analysis demonstrated that latanoprost acid was not present in any the samples collected during the skin penetration study. It is therefore justified to claim that the use of the latanoprost acid is associated with minimal risk of undesired effects related to systemic action of the molecule.

### Effects of the Invention

The results of the study indicate that latanoprost acid promotes hair regrowth in patients with androgenetic alopecia (decrease in a number of yellow dots, decrease in a percentage of follicular units with one hair, increase in a percentage of follicular units with three or more hairs, decrease in a mean percentage of vellous hairs as evaluated by trichoscopy) as well as improves the general condition of hair (increasing hair shaft thickness or hair thickness).

The results demonstrate that the invention provides not only a therapeutic, but also a cosmetic benefit with respect to reducing or preventing or treating hair loss, because various observed and measured parameters were improved in the study, in particular hair shafts thickness or hair thickness and appearance of hair.

## Claims

1. A composition comprising latanoprost acid, wherein the concentration of latanoprost acid is from 0.001 to 1%, such as 0.01%, 0.05%, 0.1%.

2. The composition according to claim 1, wherein the composition is a cosmetic composition.

3. The composition according to claim 1, wherein the composition is a pharmaceutical composition.

4. The composition according to any of the previous claims, comprising a further active ingredient.

5. Topical use of latanoprost acid as cosmetics.

6. The use according to claim 5, wherein latanoprost acid is administered to a mammal, in particular a human.

7. The use according to claim 5 or 6, wherein latanoprost acid is co-administered with another active agent.

8. The composition as defined in claim 1 for use in a method of reducing, preventing or treating hair loss and fortifying hair or promoting hair growth.

9. The composition for use as defined in claim 8 for use in a method of treating female androgenic alopecia (FAGA).

10. The composition for use according to claim 8 or 9, wherein the composition is applied 1 or 2 times per day for at least 3 months.

11. Latanoprost acid for topical use in reducing, preventing or treating hair loss and fortifying hair or promoting hair growth.

12. Latanoprost acid for use according to claim 11, wherein hair loss is caused by female androgenic alopecia (FAGA).

13. Latanoprost acid for use according to claim 11 to 12, wherein latanoprost acid is administered 1 or 2 times per day on a dry scalp for at least 3 months.

14. A cosmetic process of reducing, preventing or treating hair loss and promoting hair growth which comprises the step of applying to the skin a composition containing latanoprost acid at a concentration effective to reduce or prevent or treat the said hair loss.

## Patentansprüche

1. Zusammensetzung, umfassend Latanoprostsäure, wobei die Konzentration der Latanoprostsäure 0,001 bis 1 %, wie0,01 %, 0,05 %, 0,1 % beträgt.

2. Zusammensetzung nach Anspruch 1, wobei die Zusammensetzung eine kosmetische Zusammensetzung ist.

3. Zusammensetzung nach Anspruch 1, wobei die Zusammensetzung eine pharmazeutische Zusammensetzung ist.

4. Die Zusammensetzung nach einem der vorstehenden Ansprüche, **dadurch gekennzeichnet, dass** sie einen weiteren Wirkstoff enthält.

5. Topische Anwendung von Latanoprostsäure als Kosmetikum.

6. Verwendung nach Anspruch 5, wobei Latanoprostsäure einem Säugetier, insbesondere einem Menschen, verabreicht wird.

7. Verwendung nach Anspruch 5 oder 6, wobei Latanoprostsäure zusammen mit einem anderen Wirkstoff verabreicht wird.

8. Zusammensetzung gemäß Anspruch 1 zur Verwendung in einem Verfahren zur Verringerung, Verhinderung oder Behandlung von Haarausfall und zur Stärkung der Haare oder zur Förderung des Haarwachstums.

9. Die Zusammensetzung zur Verwendung gemäß Anspruch 8 zur Verwendung in einem Verfahren zur Behandlung der androgenetischen Alopezie der Frau (FAGA).

10. Die Zusammensetzung zur Verwendung nach Anspruch 8 oder 9, wobei die Zusammensetzung 1 oder 2 Mal pro Tag für mindestens 3 Monate angewendet wird.

11. Latanoprostsäure zur topischen Anwendung zur Verringerung, Verhinderung oder Behandlung von Haarausfall und zur Stärkung der Haare oder zur Förderung des Haarwachstums.

12. Latanoprostsäure zur Verwendung nach Anspruch 11, wobei der Haarausfall durch androgenetische Alopezie der Frau (FAGA) verursacht wird.

13. Latanoprostsäure zur Verwendung nach den Ansprüchen 11 bis 12, wobei die Latanoprostsäure mindestens 3 Monate lang 1 oder 2 mal pro Tag auf der trockenen Kopfhaut angewandt wird.

14. Kosmetisches Verfahren zur Verringerung, Verhinderung oder Behandlung von Haarausfall und zur Förderung des Haarwuchses, umfassend den Schritt des Auftragens einer Zusammensetzung die Latanoprostsäure in einer Konzentration enthält, die wirksam ist, um den besagten Haarausfall zu vermindern oder zu verhindern oder zu behandeln, auf die Haut.

## Revendications

1. Une composition comprenant de l'acide de latanoprost, dans laquelle la concentration d'acide de latanoprost est de 0,001 % à 1 %, telle que 0,01 %, 0,05 %, 0,1 %.

2. La composition selon la revendication 1, dans laquelle la composition est une composition cosmétique.

3. La composition selon la revendication 1, dans laquelle la composition est une composition pharmaceutique.

4. La composition selon l'une des revendications précédentes, comprenant un autre ingrédient actif.

5. Usage topique de l'acide de latanoprost comme produit cosmétique.

6. L'usage selon la revendication 5, dans laquelle l'acide de latanoprost est administré à un mammifère, en particulier à un être humain.

7. L'usage selon la revendication 5 ou la revendication 6, dans laquelle l'acide de latanoprost est co-administré avec un autre agent actif.

8. La composition selon la revendication 1, pour un usage dans une méthode de réduction, de prévention ou de traitement de la chute des cheveux et de fortification des cheveux ou de stimulation de la croissance des cheveux.

9. La composition telle que définie dans la revendication 8 pour un usage dans une méthode de traitement de l'alopécie androgénétique féminine.

10. La composition à utiliser selon la revendication 8 ou la revendication 9, dans laquelle la composition est appliquée 1 ou 2 fois par jour pendant au moins 3 mois.

11. L'acide de latanoprost à usage topique pour réduire, prévenir ou traiter la chute des cheveux et fortifier les cheveux ou stimuler leur croissance.

12. L'acide de latanoprost à utiliser selon la revendication 11, dans laquelle la perte de cheveux est causée par l'alopécie androgénétique féminine.

13. L'acide de latanoprost à utiliser selon les revendications 11 et 12, dans lesquelles l'acide de latanoprost est administré 1 ou 2 fois par jour sur un cuir chevelu sec pendant au moins 3 mois.

14. Un procédé cosmétique pour réduire, prévenir ou traiter la perte de cheveux et stimuler la croissance des cheveux, qui comprend l'étape consistant à appliquer sur la peau une composition contenant de l'acide de latanoprost à une concentration efficace pour réduire ou prévenir ou traiter ladite perte de cheveux.
